(19) Europäisches Patentamt
European Patent Office
Office européen des brevets

(11) Veröffentlichungsnummer: **0 041 613**
**B1**

# (12) EUROPÄISCHE PATENTSCHRIFT

(45) Veröffentlichungstag der Patentschrift: **01.02.84**

(21) Anmeldenummer: **81103429.7**

(22) Anmeldetag: **06.05.81**

(51) Int. Cl.³: **C 07 C 125/065,** C 07 C 103/375, C 07 C 155/02, A 01 N 37/22, A 01 N 47/20

## (54) Aralkylanilinderivate und Herbizide, die diese Verbindungen enthalten.

(30) Priorität: **31.05.80 DE 3020784**

(43) Veröffentlichungstag der Anmeldung: **16.12.81 Patentblatt 81/50**

(45) Bekanntmachung des Hinweises auf die Patenterteilung: **01.02.84 Patentblatt 84/5**

(84) Benannte Vertragsstaaten: **AT BE CH DE FR GB IT LI NL SE**

(56) Entgegenhaltungen:
**EP - A - 0 004 334**
**EP - A - 0 021 012**
**DE - A - 2 855 699**
**US - A - 4 162 362**

(73) Patentinhaber: **BASF Aktiengesellschaft, Carl-Bosch-Strasse 38, D-6700 Ludwigshafen (DE)**

(72) Erfinder: **Schirmer, Ulrich, Dr., Berghalde 79, D-6900 Heidelberg (DE)**
Erfinder: **Rohr, Wolfgang, Dr., In der Dreispietz 13, D-6706 Wachenheim (DE)**
Erfinder: **Wuerzer, Bruno, Dr., Ruedigerstrasse 13, D-6701 Otterstadt (DE)**

**EP 0 041 613 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Aralkylanilinderivate und Herbizide, die diese Verbindungen enthalten

Die vorliegende Erfindung betrifft neue Aralkylanilinderivate und Herbizide, welche diese Verbindungen enthalten.

Es ist bekannt, daß Aralkoxyanilinderivate eine herbizide Wirkung haben (DE-OS 2855699). Aus US-4162362 sind Diphenylmethandicarbamate und aus EP-21012 ist der N-(4-(alpha-Methylbenzyl)-phenyl)-carbaminsäureethylester bekannt. Ein Hinweis auf die herbizide Wirkung findet sich dort nicht.

Es wurde nun gefunden, daß Aralkylanilinderivate der Formel

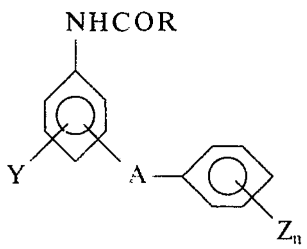

in der

R Methoxy, Ethoxy, Propoxy, sec. Butoxy, tert.-Butoxy, iso-Propoxy, Methylthio, Ethylthio, Cyclopropyl, Methyl, Ethyl, Propyl, tert. Butyl
A in 3- oder 4-Stellung zur NH-Gruppe einen gegebenenfalls durch Alkyl substituierten Alkylenrest mit 1 bis 10 C-Atomen
Y Wasserstoff, Alkyl, Halogen
Z Wasserstoff, Methyl, Ethyl, tert.-Butyl, Methoxy, Ethoxy, Hydroxy, Halogen, Trifluormethyl oder Phenyl oder eine $C_4H_4$-Kette, die mit dem Benzolring zu einem Naphthylring kondensiert ist,
n die Zahlen 1 bis 4

bedeutet, außer der Verbindung N-(4-(alpha-Methylbenzyl)-phenyl)-carbaminsäure-ethylester eine verbesserte herbizide Wirkung gegen zahlreiche wichtige unerwünschte Pflanzen haben und für verschiedene Kulturpflanzen verträglich sind.

Die in der allgemeinen Formel angeführten Reste können beispielsweise folgende Bedeutung haben:

Y Wasserstoff, Alkyl (z. B. Methyl, t-Butyl), Halogen (z. B. Fluor, Chlor, Brom)
A einen gegebenenfalls durch Alkyl substituierten Alkylenrest mit 1 bis 10 Kohlenstoffatomen (beispielsweise Methylen, Methylmethylen, Dimethylmethylen, Propylen, Hexylen, Ethylen, Methylethylen, Methylpropylen, Ethylpropylen, Butylen, Pentylen, Methylpentylen, Dimethylpropylen, Heptylen, Ethylbutylen, Trimethylpentylen).

Die neuen Verbindungen können beispielsweise nach folgendem Verfahren hergestellt werden:

Verfahren I

Ein Aralkylphenylisocyanat der Formel

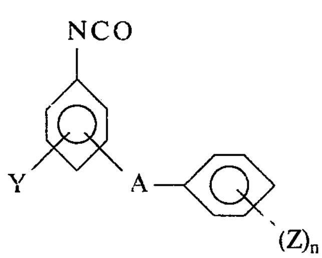

in der Y, A, $(Z)_n$ die oben genannte Bedeutung haben, wird mit einer Verbindung der Formel RH z. B. einem Alkanol oder Methylmercaptan zur Reaktion gebracht.

Nach diesem Verfahren können auch folgende Verbindungen hergestellt werden:

| Nr. | R | Y | A | $(Z)_n$ | Fp. |
|---|---|---|---|---|---|
| 4 | $OCH_3$ | H | 3-$(CH_2)_3$ | H | 35–37°C |
| 5 | $OC_2H_5$ | H | 3-$(CH_2)_3$ | H | Öl |
| 6 | O–iso $C_3H_7$ | H | 3-$(CH_2)_3$ | H | Öl |
| 7 | O–sec. $C_4H_9$ | H | 3-$(CH_2)_3$ | H | 38–41°C |
| 13 | $SCH_3$ | H | 3-$(CH_2)_3$ | H | 77–79°C |
| 17 | $C_2H_5$ | H | 3-$(CH_2)_3$ | H | 53–55°C |
| 20 | tert. $C_4H_9$ | H | 3-$(CH_2)_3$ | H | 85–86°C |
| 25 | $OCH_3$ | 4-Cl | 3-$(CH_2)_3$ | H | |
| 26 | $SCH_3$ | 4-Cl | 3-$(CH_2)_3$ | H | |
| 27 | $C_2H_5$ | 4-Cl | 3-$(CH_2)_3$ | H | |
| 28 | $OCH_3$ | 4-$CH_3$ | 3-$(CH_2)_3$ | H | |
| 29 | $SCH_3$ | 4-$CH_3$ | 3-$(CH_2)_3$ | 3-$CH_3$ | |
| 30 | $C_2H_5$ | 4-$CH_3$ | 3-$(CH_2)_3$ | 4-$CH_3$ | |
| 34 | $OCH_3$ | H | 3-$(CH_2)_3$ | 3-$OCH_3$ | 43–45°C |
| 35 | $OC_2H_5$ | H | 3-$(CH_2)_3$ | 3-$OCH_3$ | Öl |
| 36 | $SCH_3$ | H | 3-$(CH_2)_3$ | 3-$OCH_3$ | 76–78°C |
| 37 | $C_2H_5$ | H | 3-$(CH_2)_3$ | 3-$OCH_3$ | 59–61°C |
| 38 | cycl. $C_3H_5$ | H | 3-$(CH_2)_3$ | 3-$OCH_3$ | 60–62°C |
| 39 | tert. $C_4H_9$ | H | 3-$(CH_2)_3$ | 3-$OCH_3$ | 80–82°C |
| 42 | $OCH_3$ | H | 3-$(CH_2)_3$ | 3-Br | |
| 44 | $C_2H_5$ | H | 3-$(CH_2)_3$ | 4-Br | |

Fortsetzung

| Nr. | R | Y | A | $(Z)_n$ | Fp. |
|---|---|---|---|---|---|
| 48 | $SCH_3$ | H | 3-$(CH_2)_3$ | 30 $C_2H_5$ | |
| 49 | $C_2H_5$ | H | 3-$(CH_2)_3$ | 30 $C_2H_5$ | |
| 55 | $OCH_3$ | H | 3-$CH_2$ | H | 63–64°C |
| 56 | $SCH_3$ | H | 3-$CH_2$ | H | 93–94°C |
| 57 | $SC_2H_5$ | H | 3-$CH_2$ | H | 65–66°C |
| 58 | $C_2H_5$ | H | 3-$CH_2$ | H | 67–69°C |
| 59 | tert. $C_4H_9$ | H | 3-$CH_2$ | H | |
| 60 | $OCH_3$ | H | 3-$CH_2$-$CH_2$ | H | 84–85°C |
| 61 | $SCH_3$ | H | 3-$CH_2$-$CH_2$ | H | 106–108°C |
| 62 | $C_2H_5$ | H | 3-$CH_2$-$CH_2$ | H | 117–120°C |
| 63 | $OCH_3$ | H | 4-$(CH_2)_3$ | H | 76–78°C |
| 64 | $SCH_3$ | H | 4-$(CH_2)_3$ | H | 93–96°C |
| 65 | $C_2H_5$ | H | 4-$(CH_2)_3$ | H | 108–110°C |
| 66 | $OCH_3$ | 3-Cl | 4-$(CH_2)_3$ | H | |
| 67 | $SCH_3$ | 3-Cl | 4-$(CH_2)_3$ | H | |
| 68 | $C_2H_5$ | 3-Cl | 4-$(CH_2)_3$ | H | |
| 69 | $OCH_3$ | H | 3-$(CH_2)_3$ | 2-Cl | Öl |
| 70 | $SCH_3$ | H | 3-$(CH_2)_3$ | 2-Cl | 72–74°C |
| 71 | $C_2H_5$ | H | 3-$(CH_2)_3$ | 2-Cl | |
| 72 | $OCH_3$ | H | 3-$(CH_2)_3$ | 3-Cl | 44–46°C |
| 73 | $SCH_3$ | H | 3-$(CH_2)_3$ | 3-Cl | 84–85°C |

Fortsetzung

| Nr. | R | Y | A | $(Z)_n$ | Fp. |
|---|---|---|---|---|---|
| 74 | $C_2H_5$ | H | $3-(CH_2)_3$ | 3-Cl | 78–80°C |
| 75 | $OCH_3$ | H | $3-(CH_2)_3$ | 4-Cl | 83–84°C |
| 76 | $SCH_3$ | H | $3-(CH_2)_3$ | 4-Cl | 129–131°C |
| 77 | $C_2H_5$ | H | $3-(CH_2)_3$ | 4-Cl | 92–93°C |
| 78 | $OCH_3$ | H | $3-(CH_2)_3$ | 4-F | 73–75°C |
| 79 | $OC_2H_5$ | H | $3-(CH_2)_3$ | 4-F | |
| 80 | $SCH_3$ | H | $3-(CH_2)_3$ | 4-F | 108–110°C |
| 81 | $C_2H_5$ | H | $3-(CH_2)_3$ | 4-F | 93–94°C |
| 82 | $OCH_3$ | H | $3-(CH_2)_3$ | $4-OCH_3$ | 56–58°C |
| 83 | $SCH_3$ | H | $3-(CH_2)_3$ | $4-OCH_3$ | 94–96°C |
| 84 | $C_2H_5$ | H | $3-(CH_2)_3$ | $4-OCH_3$ | |
| 85 | $OCH_3$ | H | $3-(CH_2)_3$ | 3-OH | 65–67°C |
| 86 | $SCH_3$ | H | $3-(CH_2)_3$ | 3-OH | 89–91°C |
| 87 | $C_2H_5$ | H | $3-(CH_2)_3$ | 3-OH | 68–71°C |
| 88 | $OCH_3$ | H | $3-(CH_2)_3$ | $3,4\ (OCH_3)_2$ | |
| 89 | $OCH_3$ | H | $3-(CH_2)_3CH(CH_3)CH_2$ | 2,3-(CH=CH–CH=CH) | Öl |
| 90 | $SCH_3$ | H | $3-(CH_2)_3CH(CH_3)CH_2$ | 2,3-(CH=CH–CH=CH) | 96–97°C |
| 91 | $C_2H_5$ | H | $3-(CH_2)_3CH(CH_3)CH_2$ | 2,3-(CH=CH–CH=CH) | 92–94°C |
| 92 | $OCH_3$ | H | $3-(CH_2)_3$ | $2,4\ (CH_3)_2$ | |
| 93 | $SCH_3$ | H | $3-(CH_2)_3$ | $2,4\ (CH_3)_2$ | |
| 94 | $C_2H_5$ | H | $3-(CH_2)_3$ | $2,4\ (CH_3)_2$ | |

Fortsetzung

| Nr. | R | [illegible] | A | $(Z)_n$ | Fp. |
|---|---|---|---|---|---|
| 95 | $OCH_3$ | H | $3\text{-}(CH_2)_4$ | H | 28–30°C |
| 96 | $SCH_3$ | H | $3\text{-}(CH_2)_5$ | H | |
| 97 | $OCH_3$ | H | $3\text{-}CH_2CH(CH_3)CH_2$ | H | 60–61°C |
| 98 | $SCH_3$ | H | $3\text{-}CH_2CH(CH_3)CH_2$ | H | 85–87°C |
| 99 | $C_2H_5$ | H | $3\text{-}CH_2CH(CH_3)CH_2$ | H | Öl |
| 100 | $OCH_3$ | H | $3\text{-}CH_2CH(CH_3)CH_2$ | 4-F | |
| 101 | $SCH_3$ | H | $3\text{-}CH_2CH(CH_3)CH_2$ | 4-F | |
| 102 | $C_2H_5$ | H | $3\text{-}CH_2CH(CH_3)CH_2$ | 4-F | |
| 103 | $OCH_3$ | H | $3\text{-}CH_2CH(C_2H_5)CH_2$ | H | Öl |
| 104 | $SCH_3$ | H | $3\text{-}CH_2CH(C_2H_5)CH_2$ | H | Öl |
| 105 | $C_2H_5$ | H | $3\text{-}CH_2CH(C_2H_5)CH_2$ | H | 48–50°C |
| 106 | $OCH_3$ | H | $3\text{-}(CH_2)_3CH(CH_3)CH_2$ | H | 43–44°C |
| 107 | $SCH_3$ | H | $3\text{-}(CH_2)_3CH(CH_3)CH_2$ | H | 64–65°C |
| 108 | $C_2H_5$ | H | $3\text{-}(CH_2)_3CH(CH_3)CH_2$ | H | 37–39°C |
| 113 | $OCH_3$ | H | $3\text{-}(CH_2)_3$ | $2\text{-}OCH_3$ | 54–56°C |
| 114 | $SCH_3$ | H | $3\text{-}(CH_2)_3$ | $2\text{-}OCH_3$ | 68–70°C |
| 115 | $C_2H_5$ | H | $3\text{-}(CH_2)_3$ | $2\text{-}OCH_3$ | 66–68°C |
| 116 | $OCH_3$ | H | $3\text{-}(CH_2)_3$ | $3,4\text{-}Cl_2$ | |
| 117 | $SCH_3$ | H | $3\text{-}(CH_2)_3$ | $3,4\text{-}Cl_2$ | |
| 118 | $C_2H_5$ | H | $3\text{-}(CH_2)_3$ | $3,4\text{-}Cl_2$ | 79–80°C |
| 119 | $OCH_3$ | H | $3\text{-}(CH_2)_3$ | $2,6\text{-}Cl_2$ | |

Fortsetzung

| Nr. | R | Y | A | $(Z)_n$ | Fp. |
|---|---|---|---|---|---|
| 120 | $SCH_3$ | H | $3-(CH_2)_3$ | 2,6-$Cl_2$ | |
| 121 | $C_2H_5$ | H | $3-(CH_2)_3$ | 2,6-$Cl_2$ | |
| 122 | $OCH_3$ | H | $3-(CH_2)_3$ | 3-$CF_3$ | 75–76°C |
| 123 | $OC_2H_5$ | H | $3-(CH_2)_3$ | 3-$CF_3$ | |
| 124 | $SCH_3$ | H | $3-(CH_2)_3$ | 3-$CF_3$ | 97–98°C |
| 125 | $C_2H_5$ | H | $3-(CH_2)_3$ | 3-$CF_3$ | 89–90°C |
| 126 | cycl. $C_3H_5$ | H | $3-(CH_2)_3$ | 3-$CF_3$ | |
| 127 | tert. $C_4H_9$ | H | $3-(CH_2)_3$ | 3-$CF_3$ | |
| 128 | $OCH_3$ | H | $3-(CH_2)_3$ | 4 $C_6H_5$ | 75–77°C |
| 129 | $SCH_3$ | H | $3-(CH_2)_3$ | 4 $C_6H_5$ | 102–104°C |
| 130 | $C_2H_5$ | H | $3-(CH_2)_3$ | 4 $C_6H_5$ | 94–96°C |
| 131 | tert. $C_4H_9$ | H | $3-(CH_2)_3$ | 4 $C_6H_5$ | |
| 132 | $OCH_3$ | H | $3-(CH_2)_3$ | 2-$CH_3$ | Öl |
| 133 | $SCH_3$ | H | $3-(CH_2)_3$ | 2-$CH_3$ | 56–57°C |
| 134 | $C_2H_5$ | H | $3-(CH_2)_3$ | 2-$CH_3$ | 70–72°C |
| 135 | $OCH_3$ | H | $3-(CH_2)_3$ | 4-$C_2H_5$ | 80–82°C |
| 136 | $OC_2H_5$ | H | $3-(CH_2)_3$ | 4-$C_2H_5$ | |
| 137 | $SCH_3$ | H | $3-(CH_2)_3$ | 4-$C_2H_5$ | 98–100°C |
| 139 | $C_2H_5$ | H | $3-(CH_2)_3$ | 4-$C_2H_5$ | 61–63°C |
| 141 | $OCH_3$ | H | $3-(CH_2)_3$ | 4-tert. $C_4H_9$ | 57–59°C |
| 142 | $SCH_3$ | H | $3-(CH_2)_3$ | 4-tert. $C_4H_9$ | 71–72°C |

Fortsetzung

| Nr. | R | Y | A | $(Z)_n$ | Fp. |
|---|---|---|---|---|---|
| 144 | $C_2H_5$ | H | $3\text{-}(CH_2)_3$ | 4-tert. $C_4H_9$ | 75–76°C |
| 145 | tert. $C_4H_9$ | H | $3\text{-}(CH_2)_3$ | 4-tert. $C_4H_9$ | 95–97°C |
| 147 | $OCH_3$ | H | $3\text{-}(CH_2)_3$ | $4\text{-}CH_3$ | 72–74°C |
| 148 | $SCH_3$ | H | $3\text{-}(CH_2)_3$ | $4\text{-}CH_3$ | 103–105°C |
| 149 | $C_2H_5$ | H | $3\text{-}(CH_2)_3$ | $4\text{-}CH_3$ | 95–97°C |
| 150 | $OCH_3$ | H | $3\text{-}(CH_2)_3$ | 2,5 $Cl_2$ | |
| 151 | $SCH_3$ | H | $3\text{-}(CH_2)_3$ | 2,5 $Cl_2$ | |
| 152 | $C_2H_5$ | H | $3\text{-}(CH_2)_3$ | 2,5 $Cl_2$ | |
| 153 | $OCH_3$ | H | $3\text{-}(CH_2)_3$ | 2,4 $Cl_2$ | |
| 154 | $SCH_3$ | H | $3\text{-}(CH_2)_3$ | 2,4 $Cl_2$ | |
| 155 | $C_2H_5$ | H | $3\text{-}(CH_2)_3$ | 2,4 $Cl_2$ | |
| 156 | $OCH_3$ | H | $3\text{-}(CH_2)_3$ | $3\text{-}CH_3$ | 50–52°C |
| 157 | $OC_2H_5$ | H | $3\text{-}(CH_2)_3$ | $3\text{-}CH_3$ | Öl |
| 158 | $SCH_3$ | H | $3\text{-}(CH_2)_3$ | $3\text{-}CH_3$ | 81–83°C |
| 159 | $C_2H_5$ | H | $3\text{-}(CH_2)_3$ | $3\text{-}CH_3$ | 58–60°C |
| 160 | tert. $C_4H_9$ | H | $3\text{-}(CH_2)_3$ | $3\text{-}CH_3$ | 95–97°C |
| 164 | tert. $C_4H_9$ | H | $3\text{-}(CH_2)_3$ | 4-F | 101–102°C |
| 165 | cycl. $C_3H_5$ | H | $3\text{-}(CH_2)_3$ | 4-F | 96–98°C |
| 166 | tert. $C_4H_9$ | H | $3\text{-}(CH_2)_3$ | 4 $C_2H_5$ | 85–87°C |
| 167 | tert. $C_4H_9$ | H | $3\text{-}CH_2CH(CH_3)CH_2$ | H | 94–96°C |
| 168 | cycl. $C_3H_5$ | H | $3\text{-}CH_2CH(CH_3)CH_2$ | H | 73–75°C |

Fortsetzung

| Nr. | R | Y | A | $(Z)_n$ | Fp. |
|---|---|---|---|---|---|
| 169 | cycl. $C_3H_5$ | H | 3-$(CH_2)_3$ | 4 $C(CH_3)_3$ | 119–121°C |
| 170 | $CH_3$ | 4-Br | 3-$(CH_2)_3$ | H | 156–157°C |
| 171 | $OCH_3$ | 4-Br | 3-$(CH_2)_3$ | H | Öl |
| 172 | $SCH_3$ | 4-Br | 3-$(CH_2)_3$ | H | 97–100°C |
| 173 | $C_2H_5$ | 4-Br | 3-$(CH_2)_3$ | H | 103–105°C |
| 174 | $OCH_3$ | H | 4-$(CH_2)_3$ | 4 $CH_3$ | 53–55°C |
| 175 | $SCH_3$ | H | 4-$(CH_2)_3$ | 4 $CH_3$ | 99–100°C |
| 176 | $C_2H_5$ | H | 4-$(CH_2)_3$ | 4 $CH_3$ | 94–96°C |
| 183 | $OCH_3$ | H | 3-$(CH_2)_3$ | 2,4,6 $(CH_3)_3$ | |
| 184 | $SCH_3$ | H | 3-$(CH_2)_3$ | 2,4,6 $(CH_3)_3$ | |
| 185 | $C_2H_5$ | H | 3-$(CH_2)_3$ | 2,4,6 $(CH_3)_3$ | |
| 186 | $SCH_3$ | H | 3-$(CH_2)_3$ | 4-$OC_2H_5$ | 95–97°C |
| 187 | $OCH_3$ | H | 3-$(CH_2)_3$ | 4-$OC_2H_5$ | 71–73°C |
| 188 | $C_2H_5$ | H | 3-$(CH_2)_3$ | 4-$OC_2H_5$ | 90–91°C |
| 189 | $SCH_3$ | H | 4-$(CH_2)_3$ | 4-F | 98–101°C |
| 190 | $C_2H_5$ | H | 4-$(CH_2)_3$ | 4-F | 98–101°C |
| 191 | $OCH_3$ | H | 4-$CH_2$ | 4-phenyl | 133–136°C |
| 192 | $SCH_3$ | H | 4-$CH_2$ | 4-phenyl | 148–150°C |
| 193 | $C_2H_5$ | H | 4-$CH_2$ | 4-phenyl | 196–198°C |
| 194 | Cyclopropyl | H | 3-$(CH_2)_3$ | 2-$CH_3$ | 99–101°C |
| 195 | Cyclopropyl | H | 3-$(CH_2)_3$ | 3-Cl | 88–89°C |

Fortsetzung

| Nr. | R | Y | A | $(Z)_n$ | Fp. |
|---|---|---|---|---|---|
| 196 | Cyclopropyl | H | 4-$(CH_2)_3$ | 4-$CH_3$ | 113–115°C |
| 198 | $SCH_3$ | H | 4-$(CH_2)_4$ | H | 95–97 |
| 199 | $C_2H_5$ | H | 3-$(CH_2)_4$ | H | 50–52°C |
| 200 | $SCH_3$ | H | 4-$(CH_2)_2$ | H | 120–122 |
| 201 | $C_2H_5$ | H | 4-$(CH_2)_2$ | H | 108–110°C |

Die Anwendung der Wirkstoffe erfolgt z. B. in Form von direkt versprühbaren Lösungen, Pulvern, Suspensionen — auch hochprozentige wässerige, ölige oder sonstige Suspensionen — oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken.

Beispiel 1

8,14 g tert.-Butanol werden zu einer Lösung aus 23,7 g 3-(3-Phenylpropyl)-phenylisocyanat und 1 Tropfen Triethylamin in 200 ml absolutem Toluol gegeben. Nach fünftägigem Stehen wird das Lösungsmittel abdestilliert. Man erhält 24,5 g einer weißen kristallinen Substanz vom Schmelzpunkt 83—85°C und folgender Strukturformel (Wirkstoff Nr. 1)

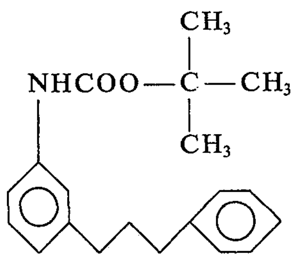

Beispiel 2

Zu einer Mischung aus 15,8 g 3-(3-Phenylpropyl)-anilin, 8,5 $NaHCO_3$ und 200 ml THF tropft man bei 20°C 7,9 g Cyclopropancarbonsäurechlorid. Man läßt über Nacht rühren, filtriert ab, destilliert das Lösungsmittel ab und verrührt mit Petrolether. Nach dem Absaugen und Trocknen erhält man 19,3 g einer Verbindung, die bei 96—99°C schmilzt und folgende Strukturformel hat (Wirkstoff Nr. 2):

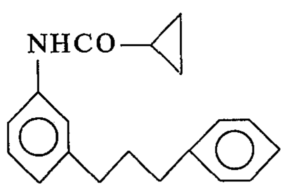

Beispiel 3

253 g 1-Phenyl-3-(3-Nitrophenyl)-propen-2-on-1 und 10 g 10% Palladium auf Tierkohle werden in einer Mischung aus 2,5 l Eisessig und 430 g Acetanhydrid bei 65 bis 70°C so lange hydriert (Wasserstoffdruck, 1,1 bar), bis keine Wasserstoffaufnahme mehr erfolgt. Nach dem Filtrieren wird der größte Teil Essigsäure abdestilliert. Das verbleibende Öl wird in Wasser eingerührt und abgesaugt.

Man erhält 239 g einer weißen kristallinen Substanz vom Schmelzpunkt 71 bis 73°C mit folgender Strukturformel (Wirkstoff Nr. 3)

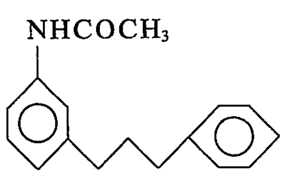

Zur Herstellung von direkt versprühbaren Lösungen, Emulsionen, Pasten und Öldispersionen kommen Mineralölfraktionen von mittlerem mit hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z. B. Dimethylformid, Dimethylsulfoxid, N-Methylpyrrolidon, Wasser usw. in Betracht.

Wässerige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel

gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen:
Alkali-, Erdalkali-, Ammoniumsalze von Ligninsulfonsäure,
Naphthalinsulfonsäuren, Phenolsulfonsäuren, Alkylarylsulfonate,
Alkylsulfate, Alkylsulfonate,
Alkali- und Erdalkalisalze der Dibutylnapthalinsulfonsäure,
Laurylethersulfat, Fettalkoholsulfate,
fettsaure Alkali- und Erdalkalisalze,
Salze sulfatierter Hexadecanole, Heptadecanole, Octadecanole,
Salze von sulfatiertem Fettalkoholglykolether,
Kondensationsprodukte von sulfoniertem Naphthalin und Naphthalinderivaten mit Formaldehyd,
Kondensationsprodukte des Naphthalins bzw. der Naphthalinsulfonsäuren mit Phenol und Formaldehyd,
Polyoxyethylen-octylphenolether,
ethoxyliertes Isooctylphenol-, Octylphenol-, Nonylphenol,
Alkylphenolpolyglykolether, Tributylphenylpolyglykolether,
Alkylarylpolyetheralkohole, Isotridecylalkohol,
Fettalkoholethylenoxid-Kondensate, ethoxyliertes Rizinusöl,
Polyoxyethylenalkylether, ethoxyliertes Polyoxypropylen,
Laurylalkoholpolyglykoletheracetal, Sorbitester, Lignin,
Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz und Nußschalenmehl, Cellulosepulver und andere feste Trägerstoffe.

Die Formulierungen enthalten zwischen 0,1 und 95 Gewichtsprozent Wirkstoff, vorzugsweise zwischen 0,5 und 90 Gewichtsprozent.

Die Wirkstoffe werden angewendet, beispielsweise durch Gießen, Streuen, Stäuben, Spritzen oder Sprühen auf die Pflanzen oder den Boden, durch Injizieren oder Bestreichen von Pflanzen oder durch Einbringen in das Bewässerungswasser.

## Beispiel I

Man vermischt 90 Gewichtsteile der Verbindung 1 mit 10 Gewichtsteilen N-Methyl-alpha-pyrrolidon und erhält eine Lösung, die zur Anwendung in Form kleinster Tropfen geeignet ist.

## Beispiel II

10 Gewichtsteile der Verbindung 2 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-mono-ethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

## Beispiel III

20 Gewichtsteile der Verbindung 2 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

Beispiel IV

20 Gewichtsteile der Verbindung 3 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht.

Beispiel V

80 Gewichtsteile des Wirkstoffs 1 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Beispiel VI

5 Gewichtsteile der Verbindung 1 werden mit 95 Gewichtsteilen feinteiligem Kaolin innig vermischt. Man erhält auf diese Weise ein Stäubemittel, das 95 Gewichtsprozent des Wirkstoffs enthält.

Beispiel VII

30 Gewichtsprozent der Verbindung 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel VIII

40 Gewichtsteile des Wirkstoffs 1 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wäßrige Dispersion.

Beispiel IX

20 Teile des Wirkstoffs 1 werden mit 12 Teilen Calciumsalz der Dodecylbenzolsulfonsäure, 8 Teile Fettalkohol-polyglykolether, 2 Teilen Natriumsalz eines Phenolsulfonsäure-harnstoff-formaldehyd-kondensats und 68 Teilen eines paraffinischen Mineralöls innig vermischt. Man erhält eine stabile ölige Dispersion.

Die Applikation kann im Vorauflaufverfahren oder bei Nachauflaufanwendung erfolgen. Vorzugsweise werden die neuen Wirkstoffe nach dem Auflaufen der unerwünschten Pflanzen, sowohl auf Kulturflächen als auch auf unbebautem Land, ausgebracht. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen ans Wirkstoff betragen je nach Jahreszeit und Wachstumsstadium 0,1 bis 15 kg/ha und mehr.

Der Einfluß von Vertretern der neuen herbiziden Aralkylanilinderivate auf das Wachstum von erwünschten und unerwünschten Pflanzen wird anhand von Gewächshausversuchen vorgeführt:

Als Kulturgefäße dienten Plastikblumentöpfe mit 300 $cm^3$ Inhalt und lehmiger Sand mit etwa 1,5% Humus als Substrat. Die Samen der Testpflanzen wurden nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt. Bei dieser Applikationsmethode handelt es sich um eine Aufwandmenge entsprechend 3,0 kg Wirkstoff/ha. Nach dem Aufbringen der Mittel wurden die Gefäße leicht beregnet, um Keimung und Wachstum in Gang zu bringen. Danach deckte man die Gefäße mit durchsichtigen Plastikhauben ab, bis die Pflanzen angewaschen waren. Diese Abdeckung bewirkte ein gleichmäßiges Keimen der Testpflanzen, sofern dies nicht durch die Wirkstoffe beeinträchtigt wurde. Zum Zwecke der Nachauflaufbehandlung zog man die Testpflanzen je nach Wuchsform erst bis zu einer Wuchshöhe von 3 bis 10 cm an und behandelte sie danach. Zur Nachauflaufbehandlung wurden entweder direkt gesäte

und in den gleichen Gefäßen aufgewachsene Pflanzen ausgewählt, oder sie wurden erst als Keimpflanzen getrennt angezogen und einige Tage vor der Behandlung in die Versuchsgefäße verpflanzt. Die Aufwandmenge für die Nachauflaufbehandlung variiert je nach Wirkstoff. Sie betrugen im Einzelfall 0,25 oder 0,5 oder 1,0 kg/ha Wirkstoff. Als Vergleichsmittel wurden die bekannten Wirkstoffe (DE-OS 2 855 699) N-(3-(2-Phenylethoxy)-phenyl)-O-methylcarbamat (A) zu 0,25 kg/ha, N-(3-(2-Phenylethoxy)-phenyl)-O-ethylcarbamat (B) zu 0,5 kg/ha, N-(3-(2-Phenylethoxy)-phenyl-propionamid (C) zu 0,5 kg/ha und N-(3-(2-(4-Chlorphenyl)-ethoxy)-phenyl)-O-methylcarbamat (D) zu 0,25 kg/ha gewählt. Eine Abdeckung unterblieb bei der Nachauflaufbehandlung. Die Aufstellung der Versuche erfolgte im Gewächshaus, wobei für wärmeliebende Arten wärmere Bereiche (20 bis 30°C) und für solche gemäßigter Klimate 15 bis 25°C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100.

Dabei bedeutet 0 keine Schädigung oder normaler Auflauf und 100 kein Aufgang der Pflanzen bzw. völlige Zerstörung zumindest der oberirdischen Teile.

Die Ergebnisse zeigen, daß bei Nachauflaufanwendung die neuen Verbindungen bei einer Reihe von unerwünschten Pflanzen eine bessere herbizide Aktivität haben als die Vergleichsmittel. Daneben gibt es einige erfindungsgemäße Verbindungen, welche darüber hinaus auch eine günstigere Verträglichkeit gegenüber bestimmten Kulturpflanzen aufweisen. Bei Vorauflaufanwendung wird ebenfalls eine herbizide Wirkung beobachtet.

Sind gewisse Kulturpflanzen gegenüber den Wirkstoffen etwas empfindlich, so können auch Ausbringungstechniken angewandt werden, bei welchen die herbiziden Mittel mit Hilfe der Spritzgeräte so geleitet werden, daß die Blätter empfindlicher Kulturpflanzen nach Möglichkeit nicht getroffen werden, während sie auf die Blätter darunter wachsender unerwünschter Pflanzen oder die unbedeckte Bodenfläche gelangen (post directed, lay-by). In Anbetracht der guten Verträglichkeit und der Vielseitigkeit der Applikationsmethoden können die erfindungsgemäßen Herbizide noch in einer weiteren großen Zahl von Kulturpflanzen zur Beseitigung unerwünschten Pflanzenwuchses eingesetzt werden. Die Aufwandmengen können dabei zwischen 0,1 und 15 kg/ha und mehr schwanken.

In Betracht kommen beispielsweise die folgenden Kulturen:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fodder beets |
| Beta vulgaris spp. esculenta | Rote Rübe | table beets, red beets |
| Brassica napus var. napus | Raps | rape seed |
| Brassica napus var. napobrassica | Kohlrübe | |
| Brassica napus var. rapa | Weiße Rübe | turnips |
| Brassica rapa var. silvestris | Rübsen | |
| Camellia sinensis | Teestrauch | tea plants |
| Carthamus tinctorius | Saflor-Färberdistel | safflower |
| Carya illinoinensis | Pekannußbaum | pecan trees |

Fortsetzung

| Botanischer Name | Deutscher Name | Englischer Name |
|---|---|---|
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagras | Bermudagrass in turfs and lawns |
| Daucus carota | Möhre | carrots |
| Elaeis guineensis | Ölpalme | oil palms |
| Fragaria vesca | Erdbeere | strawberries |
| Glyoine max | Sojabohne | soybeans |
| Gossypium hirsutum (Gossypium arboreum Gossypium herbaceum Gossypium vitifolium) | Baumwolle | cotton |
| Helianthus annuus | Sonnenblume | sunflowers |
| Helianthus tuberosus | Topinambur | |
| Hevea brasiliensis | Parakatuschukbaum | rubber plants |
| Hordeum vulgare | Gerste | barley |
| Humulus lupulus | Hopfen | hop |
| Ipomoea batatas | Süßkartoffeln | sweet potato |
| Juglans regia | Walnußbaum | walnut trees |
| Lactua sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |
| Manihot esculenta | Maniok | cassava |
| Medicago sativa | Luzerne | alfalfa (lucerne) |
| Metha piperita | Pfefferminze | peppermint |

Fortsetzung

| Botanischer Name | Deutscher Name | Englischer Name |
|---|---|---|
| Musa spp. | Obst- und Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beans, dry beans |
| Pennisetum glaucum | Perl- oder Rohrkolbenhirse | |
| Petroselinum crispum spp. tuberosum | Wurzelpetersilie | parsley |
| Picea abies | Rotfichte | Norway spruce |
| Abies alba | Weißtanne | fire |
| Pinus spp. | Kiefer | pine trees |
| Pisum sativum | Gartenerbse | English peas |
| Prunus avium | Süßkirsche | cherry trees |
| Prunus domestica | Pflaume | plum trees |
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sasamum indicum | Sesam | Sesame |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |

Fortsetzung

| Botanischer Name | Deutscher Name | Englischer Name |
|---|---|---|
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preiselbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn maize |

Zur Verbreiterung des Wirkungsspektrums und zur Erzielung synergistischer Effekte können die neuen Aralkylanilinderivate sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4 H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht. Eine Reihe von Wirkstoffen, welche zusammen mit den neuen Verbindungen für verschiedenste Anwendungsbereiche sinnvolle Mischungen ergeben, werden beispielhaft aufgeführt:

5-Amino-4-chlor-2-phenyl-3(2 H)-pyridazinon
5-Amino-4-brom-2-phenyl-3(2 H)-pyridazinon
5-Amino-4-chlor-2-cyclohexyl-3(2 H)-pyridazinon
5-Amino-4-brom-2-cyclohexyl-3(2 H)-pyridazinon

5-Methylamino-4-chlor-2-(3.trifluormethylphenyl-3(2 H)-pyridazinon
5-Methylamino-4-chlor-2-(3.$\alpha,\alpha,\beta,\beta$-tetrafluorethoxyphenyl)-3(2 H)-pyridazinon
5-Dimethylamino-4-chlor-2-phenyl-3(2 H)-pyridazinon
4,5-Dimethoxy-2-phenyl-3(2 H)-pyridazinon
4,5-Dimethoxy-2-cyclohexyl-3(2 H)-pyridazinon
4,5-Dimethoxy-2-(3.trifluormethylphenyl)-3(2 H)-pyridazinon
5-Methoxy-4-chlor-2-(3.trifluormethylphenyl)-3(2 H)-pyridazinon
5-Amino-4-brom-2-(3.methylphenyl)-3(2 H)-pyridazinon

3-(1-Methylethyl)-1 H-2,1,3-benzothiadiazin-4(3 H)-on-2,2-dioxid und Salze
3-(1-Methylethyl)-8-chlor-1 H-2,1,3-benzothiadiazin-4(3 H)-on-2,2-dioxid und Salze
3-(1-Methylethyl)-8-fluor-1 H-2,1,3-benzothiadiazin-4(3 H)-on-2,2-dioxid und Salze
3-(1-Methylethyl)-8-methyl-1 H-2,1,3-benzothiadiazin-4(3 H)-on-2,2-dioxid und Salze
1-Methoxymethyl-3-(1-methylethyl-2,1,3-benzothiadiazin-4(3 H)-on-2,2-dioxid
1-Methoxymethyl-8-chlor-3-(1-methylethyl)-2,1,3-benzothiadiazin-4(3 H)-on-2,2-dioxid
1-Methoxymethyl-8-fluor-3-(1-methylethyl)-2,1,3-benzothiadiazin-4(3 H)-on-2,2-dioxid
1-Cyan-8-chlor-3-(1-methylethyl)-2,1,3-benzothiadiazin-4(3 H)-on-2,2-dioxid
1-Cyan-8-fluor-3-(1-methylethyl)-2,1,3-benzothiadiazin-4(3 H)-on-2,2-dioxid
1-Cyan-8-methyl-3-(1-methylethyl)-2,1,3-benzothiadiazin-4(3 H)-on-2,2-dioxid
1-Cyan-3-(1-methylethyl)-2,1,3-benzothiadiazin-4(3 H)-on-2,2-dioxid
1-Azidomethyl-3-(1-methylethyl)-2,1,3-benzothiadiazin-4(3 H)-on-2,2-dioxid
3-(1-Methylethyl)-1 H-pyridino-[3,2-e]2,1,3-thiadiazin-(4)-on-2,2-dioxid

N-(1-Ethylpropyl)-2,6-dinitro-3,4-dimethylanilin
N-(1-Methyläthyl)-N-ethyl-2,6-dinitro-4-trifluormethylanilin
N-n.Propyl-N-$\beta$-chlorethyl-2,6-dinitro-4-trifluormethylanilin
N-n.Propyl-N-cyclopropylmethyl-2,6-dinitro-4-trifluormethyl-anilin

N-Bis-(n.propyl)-2,6-dinitro-3-amino-4-trifluormethylanilin
N-Bis-(n.propyl)-2,6-dinitro-4-methyl-anilin
N-Bis-(n.propyl)-2,6-dinitro-4-methylsulfonyl-anilin
N-Bis-(n.propyl)-2,6-dinitro-4-aminosulfonyl-anilin
Bis-(β-chlorethyl)-2,6-dinitro-4-methyl-anilin
N-Ethyl-N-(2-methylallyl)-2,6-dinitro-4-trifluormethyl-anilin

N-Methylcarbaminsäure-3,4-dichlorbenzylester
N-Methylcarbaminsäure-2,6-di-tert.butyl-4-methylphenylester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
3'-N-Isopropyl-carbamoyloxy-propionsäureanilid

Ethyl-N-(3-(N'-phenylcarbamoyloxy)-phenyl)-carbamat
Methyl-N-(3-(N'-methyl-N'-phenylcarbamoyloxy)-phenyl)-carbamat
Isopropyl-N-(3-(N'-ethyl-N'-phenylcarbamoyloxy)-phenyl)-carbamat
Methyl-N-(3-(N'-3-methylphenylcarbamoyloxy)-phenyl)-carbamat
Methyl-N-(3-(N'-4-fluorphenylcarbamoyloxy)-phenyl)-carbamat
Methyl-N-(3-(N'-3-chlor-4-fluorphenylcarbamoyloxy)-phenyl)-carbamat
Ethyl-N-(3-(N'-3-chlor-4-fluorphenylcarbamoyloxy)-phenyl)-carbamat
Ethyl-N-(3-(N'-3,4-difluorphenylcarbamoyloxy)-phenyl)-carbamat
Methyl-N-(3-N'-3,4-difluorphenylcarbamoyloxy)-phenyl)-carbamat

N-3-(4-Fluorphenoxycarbonylamino)-phenylcarbaminsäure-methylester
N-3-(2-Methylphenoxycarbonylamino)-phenylcarbaminsäure-ethylester
N-3-(4-Fluorphenoxycarbonylamino)-phenylthiocarbaminsäure-methylester
N-3-(2,4,5-Trimethylphenoxycarbonylamino)-phenylthiocarbaminsäure-methylester
N-3-(Phenoxycarbonylamino)-phenylthiolcarbaminsäure-methylester

N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-ethyl-5-isoxazolyl-methylester
N,N-Di-sec.butyl-thiolcarbaminsäure-ethylester
N,N-Di-sec.butyl-thiolcarbaminsäure-benzylester
N-Ethyl-N-cyclohexyl-thiolcarbaminsäure-ethylester
N-Ethyl-N-bicyclo-[2,2,1]-heptyl-thiolcarbaminsäure-ethylester
S-(2,3-Dichlorallyl)-(2,2,4-trimethyl-azetidin)-1-carbothiolat
S-(2,3,3-Trichlorallyl)-(2,2,4-trimethyl-azetidin)-1-carbothiolat
S-Ethyl-hexahydro-1-H-azepin-1-carbothiolat
S-Benzyl-3-methylhexahydro-1-H-azepin-1-carbothiolat
S-Benzyl-2,3-dimethylhexahydro-1-H-azepin-1-carbothiolat
S-Ethyl-3-methylhexahydro-1-H-azepin-1-carbothiolat
N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
α,α-Dichlorpropionsäure-Natriumsalz
α,α-Dichlorbuttersäure-Natriumsalz
α,α,β,β-Tetrafluorpropionsäure-Natriumsalz
α-Methyl,α,β-dichlorpropionsäure-Natriumsalz
α-Chlor-β-(4-chlorphenyl)-propionsäure-methylester
α,β-Dichlor-β-phenylpropionsäure-methylester
Benzamido-oxy-essigsäure
2,3,5-Triiodbenzoesäure (Salze, Ester, Amide)

2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephthalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäuremethylester
2-[4-(2'-Chlor-4'-trifluorphenoxy)-phenoxy]-propionsäure-Natriumsalz
2-[4-(3',5'-Dichlorpyridyl-2-oxy)-phenoxy]-propionsäure-Natriumsalz

2-(N-Benzoyl-3,4-dichlorphenylamino)-propionsäureethylester
2-(N-Benzoyl-3-chlor-4-fluorphenylamino)-propionsäure-methylester
2-(N-Benzoyl-3-chlor-4-fluorphenylamino)-propionsäure-isopropylester

2-Chlor-4-ethylamino-6-isopropylamino-1,3,5-triazin
2-Chlor-4-ethylamino-6-(amino-2'-propionitril)-1,3,5-triazin
2-Chlor-4-ethylamino-6-2-methoxypropyl-2-amino-1,3,5-triazin
2-Chlor-4-ethylamino-6-butin-1-yl-2-amino-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin

2-Azido-4-methylamino-6-isopropylamino-1,3,5-triazin
2-Methylthio-4-ethylamino-6-isopropylamino-1,3,5-triazin
2-Methylthio-4-ethylamino-6-tert.butylamino-1,3,5-triazin
2-Methylthio-4,6-bisethylamino-1,3,5-triazin
2-Methylthio-4,6-bisisopropylamino-1,3,5-triazin

2-Methoxy-4-ethylamino-6-isopropylamino-1,3,5-triazin
2-Methoxy-4,6-bisethylamino-1,3,5-triazin
2-Methoxy-4,6-bisiisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion

3-tert.Butyl-5-chlor-6-methyluracil
3-tert.Butyl-5-brom-6-methyluracil
3-Isopropyl-5-brom-6-methyluracil
3-sec.Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethyluracil

2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol
1-Allyloxy-1-(4-bromphenyl)-2-[1',2',4'-triazolyl-(1')]-ethan (Salze)
1-(4-Chlorphenoxy-3,3-dimethyl-1(1 H-1,2,4-triazol-1-yl)-2-butanon
N,N-Diallylchloracetamid
N-Isopropyl-2-chloracetanilid
N-(1-Methyl-propin-2-yl)-2-chloracetanilid

2-Methyl-6-ethyl-N-(propargyl)-2-chloracetanilid
2-Methyl-6-ethyl-N-(ethoxymethyl)-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracetanilid
2-Methyl-6-ethyl-N-(4-methoxypyrazol-1-yl-methyl)-2-chloracetanilid
2-Methyl-6-ethyl-N-(pyrazol-1-yl-methyl)-2-chloracetanilid

2,6-Dimethyl-N-(pyrazol-1-yl-methyl)-2-chloracetanilid
2,6-Dimethyl-N-(4-methylpyrazol-1-yl-methyl)-2-chloracetanilid
2,6-Dimethyl-N-(1,2,4-triazol-1-yl-methyl)-2-chloracetanilid
2,6-Dimethyl-N-(3,5-dimethylpyrazol-1-yl-methyl)-2-chloracetanilid
2,6-Dimethyl-N-(1,3-dioxolan-2-yl-methyl)-2-chloracetanilid
2,6-Dimethyl-N-(2-methoxyethyl)-2-chloracetanilid
2,6-Dimethyl-N-(isobutoxymethyl)-2-chloracetanilid
2,6-Diethyl-N-(methoxymethyl)-2-chloracetanilid
2,6-Diethyl-N-(n-butoxymethyl)-2-chloracetanilid
2,6-Diethyl-N-(ethoxycarbonylmethyl)-2-chloracetanilid
2,3,6-Trimethyl-N-(pyrazol-1-yl-methyl)-2-chloracetanilid
2,3-Dimethyl-N-(isopropyl)-2-chloracetanilid
2,6-Diethyl-N-(2-n-propoxyethyl)-2-chloracetanilid

2-(2-Methyl-4-chlorphenoxy)-N-methoxy-acetamid
2-(α-Naphtoxy)-N,N-diethylpropionamid
2,2-Diphenyl-N,N-dimethylacetamid
α-(3,4,5-Tribrompyrazol-1-yl)-N,N-dimethylpropionamid
N-(1,1-Dimethylpropinyl)-3,5-dichlorbenzamid
N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyltrifluormethan-sulfonanilid
5-Acetamido-4-methyl-trifluormethan-sulfonanilid

2-Propionyl-amino-4-methyl-5-chlor-thiazol
O-(Methylsulfonyl)-glykolsäure-N-ethoxymethyl-2,6-dimethylanilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Diiod-4-hydroxy-benzonitril (Salze)
3,5-Dibrom-4-hydroxy-O-2,4-dinitrophenylbenzaldoxim (Salze)
3,5-Dibrom-4-hydroxy-O-2-cyan-4-nitrophenylbenzaldoxim (Salze)
Pentachlorphenol-Natriumsalz
2,4-Dichlorphenyl-4′-nitrophenylether
2,4,6-Trichlorphenyl-4′-nitrophenylether
2-Fluor-4,6-dichlorphenyl-4′-nitrophenylether
2-Chlor-4-trifluormethylphenyl-4′-nitrophenylether
2,4′Dinitro-4-trifluormethyl-diphenylether
2,4-Dichlorphenyl-3′-methoxy-4′-nitro-phenylether
2-Chlor-4-trifluormethylphenyl-3′-ethoxy-4′-nitro-phenylether
2-Chlor-4-trifluormethylphenyl-3′-carboxy-4′-nitro-phenylether (Salze)
2,4-Dichlorphenyl-3′-methoxycarbonyl-4′-nitro-phenylether
2-(3,4-Dichlorphenyl)-4-methyl-1,2,4-oxadiazolidin-3,5-dion
2-(3-tert.Butylcarbamoyl-oxyphenyl)-4-methyl-1,2,4-oxadiazolidin-3,5-dion
2-(3-iso-Propylcarbamoyl-oxyphenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2,6}$$0,^{8,11}$]-dodeca-3,9-dien
2-Ethoxy-2,3-dihydro-3,3-dimethyl-5-benzofuranyl-methansulfonat
2-Ethoxy-2,3-dihydro-3,3-dimethyl-5-benzofuranyl-dimethylaminosulfonat
2-Ethoxy-2,3-dihydro-3,3-dimethyl-5-benzofuranyl-(N-methyl-N-acetyl)-aminosulfonat
3,4-Dichlor-1,2-benzisothiazol
N-4-Chlorphenyl-allylbernsteinsäureimid
2-Methyl-4,6-dinitrophenol (Salze, Ester)
2-sec.Butyl-4,6-dinitrophenol (Salze)
2-sec.Butyl-4,6-dinitrophenol-acetat
2-tert.Butyl-4,6-dinitrophenol-acetat

2-tert.Butyl-4,6-dinitrophenol (Salze)
2-tert.Butyl-5-methyl-4,6-dinitrophenol (Salze)

2-tert.Butyl-5-methyl-4,6-dinitrophenol-acetat

2-sec.Amyl-4,6-dinitrophenol (Salze, Ester)
1-(α,α-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff
1-(4-Chlorphenyl)-1-benzoyl-3,3-dimethyl-harnstoff
1-(4-Chlorphenyl)-3,3-dimethyl-harnstoff
1-(4-Chlorphenyl)-3-methyl-3-butin-1-yl-3-harnstoff
1-(3,4-Dichlorphenyl)-3,3-dimethyl-harnstoff
1-(3,4-Dichlorphenyl)-1-benzoyl-3,3-dimethyl-harnstoff
1-(3,4-Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(3-α,α,β,β-Tetrafluorethoxyphenyl)-3,3-dimethyl-harnstoff

1-(3-tert.-Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4(4'-Chlorphenoxy)-phenyl]-3,3-dimethyl-harnstoff
1-[4(4'-methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethylharnstoff
Imidazolidin-2-on-1-carbonsäure-iso-butylamid
1,2-Dimethyl-3,5-diphenylpyrazolium-methylsulfat
1,2,4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[4-methylphenyl)-sulfonyl-oxy]-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1'-Dimethyl-4,4'-dipyridylium-di-(methylsulfat)
1,1'-Di(3,5-dimethylmorpholin-carbonylmethyl)-4,4'-dipyridylium-dichlorid
1,1'-Ethylen-2,2'-dipyridylium-dibromid
3-[1(N-Ethoxyamino)-propyliden]-6-ethyl-3,4-dihydro-2-H-pyran-2,4-dion
3-[1-(N-Allyloxyamino)-propyliden]-6-ethyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino)-butyliden]-5,5-dimethylcyclohexan-1,3-dion (Salze)
2-[1-(N-Allyloxyamino)-butyliden]-5,5-dimethyl-4-methoxycarbonyl-cyclohexan-1,3-dion (Salze)
2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2,4,5-Trichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)
α-Naphthoxyessigsäuremethylester
2-(2-Methylphenoxy)-propionsäure (Salze, Ester, Amide)
2-(4-Chlorphenoxy)-propionsäure (Salze, Ester, Amide)

2-(2,4-Dichlorphenoxy)-propionsäure (Salze, Ester, Amide)
2-(2,4,5-Trichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
Cyclohexyl-3-(2,4-dichlorphenoxy)-acrylat
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)
Gibellerinsäure (Salze)
Dinatrium-methylarsonat
Mononatriumsalz der Methylarsonsäure
N-Phosphon-methyl-glycin (Salze)
N,N-Bis-(phosphormethyl)-glycin (Salze)
2-Chlorethanphosphonsäure-2-chlorethylester
Ammonium-ethyl-carbamoyl-phosphonat
Di-n-butyl-1-n-butylamino-cyclohexyl-phosphonat
Trithiobutylphosphit
O,O-Diisopropyl-5-(2-benzosulfonylamino-ethyl)-phosphordithioat
2,3-Dihydro-5,6-dimethyl-1,4-dithiin-1,1,4,4-tetraoxid
5-tert.Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N-dimethylhydrazid (Salze)
(2-Chlorethyl)-trimethyl-ammoniumchlorid
(2-Methyl-4-phenylsulfonyl)-trifluormethansulfonanilid
1,1-Dimethyl-4,6-diisopropyl-5-indanylethylketon
Natriumchlorat
Ammoniumrhodanid
Calciumcyanamid
2-Chlor-4-trifluormethylphenyl-3'-ethoxycarbonyl-4'-nitrophenylether

1-(4-Benzyloxyphenyl)-3-methyl-3-methoxyharnstoff
2-[1-(2,5-Dimethylphenyl)-ethylsulfonyl]-pyridin-N-oxid
1-Acetyl-3-anilino-4-methoxycarbonyl-5-methylpyrazol
3-Anilino-4-methoxycarbonyl-5-methylpyrazol
3-tert.Butylamino-4-methoxycarbonyl-5-methylpyrazol
N-Benzyl-N-isopropyl-trimethylacetamid
2-[4-(4'-Chlorphenoxymethyl)-phenoxy]-propionsäuremethylester
2-[4-(5'-Brompyridyl-2-oxy)-phenoxy]-propionsäureethylester
2-[4-(5'-Jodpyridyl-2-oxy)-phenoxy]-propionsäureethylester
2-[4-(5'-Jodpyridyl-2-oxy)-phenoxy]-propionsäure-n.-butylester
2-Chlor-4-trifluormethylphenyl-3'-(2-fluorethoxy)-4'-nitro-phenylether
2-Chlor-4-trifluormethylphenyl-3-(ethoxycarbonyl)methylthio-4-nitrophenylether
2,4,6-Trichlorphenyl-3(ethoxycarbonyl)methylthio-4-nitro-phenylether
2-[1-(N-ethoxamino)-butyliden]-5-(2-ethylthiopropyl)-3-hydroxy-cyclohexen-(2)-on-(1) (Salze)
2-[1-(N-ethoxamino)-butyliden]-5-(2-phenylthiopropyl)-3-hydroxy-cyclohexen-(2)-on-(1) (Salze)
4-[4-(4'-Trifluormethyl)-phenoxy]-penten-2-carbonsäureethylester
2-Chlor-4-trifluormethyl-3'-methoxycarbonyl-4'-nitrophenylether
2,4-Dichlorphenyl-3'-carboxy-4'-nitrophenylether (Salze)
4,5-Dimethoxy-2-(3-$\alpha,\alpha,\beta$-trifluor-$\beta$-bromethoxyphenyl)-3-(2 H)-pyridazinon
2,4-Dichlorphenyl-3'-ethoxy-ethoxy-ethoxy-4'-nitrophenylether
2,3-Dihydro-3,3-dimethyl-5-benzofuranyl-ethansulfonat
N-[4-Methoxy-6-methyl-1,3,5-triazin-2-yl-aminocarbonyl]-2-chlorbenzolsulfonamid
1(3-Chlor-4-ethoxyphenyl)-3,3-dimethylharnstoff
2-Methyl-4-Chlorphenoxy-thioessigsäureethylester
2-Chlor-3,5-dijod-4-acetoxy-pyridin

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

Bei der Prüfung auf selektive herbizide Wirkung bei Nachauflaufanwendung im Gewächshaus bei Aufwandmengen von 0,25 kg Wirkstoff/ha zeigen die neuen Verbindungen Nr. 4 und Nr. 13 eine bessere herbizide Wirkung als der bekannte Wirkstoff A.

Ebenfalls bei 0,25 kg Wirkstoff/ha und bei Nachauflaufanwendung im Gewächshaus erbringt der neue Wirkstoff Nr. 148 eine bessere herbizide Wirkung und eine bessere Verträglichkeit als der bekannte Wirkstoff D.

Bei der Prüfung auf selektive herbizide Wirkung bei Nachauflaufanwendung im Gewächshaus bei Aufwandmengen von 0,5 kg Wirkstoff/ha zeigen die neuen Verbindungen Nr. 17, 149 und 77 eine bessere herbizide Aktivität als der bekannte Wirkstoff C.

Gleichfalls bei der selektiven Unkrautbekämpfung bei Nachauflaufanwendung im Gewächshaus bei Aufwandmengen von 0,5 kg Wirkstoff/ha weist der neue Wirkstoff Nr. 5 eine bessere selektive herbizide Wirkung auf als der bekannte Wirkstoff B.

Bei der Prüfung der herbiziden Wirkung bei Vorauflaufanwendung im Gewächshaus bei Aufwandmengen von 3,0 kg Wirkstoff/ha zeigen die neuen Wirkstoffe Nr. 1, 2, 4, 7, 20, 55, 58, 78, 81, 99, 129, 142, 144, 147, 157 und 159 eine zum Teil sehr gute herbizide Wirkung.

Bei der Prüfung der herbiziden Wirkung bei Nachauflaufanwendung im Gewächshaus bei Aufwandmengen von 0,125 und 0,5 kg Wirkstoff je ha zeigten die Wirkstoffe 36 und 175 eine gute herbizide Wirkung.

Bei der Prüfung der selektiven herbiziden Wirkung bei Nachauflaufanwendung im Gewächshaus bei einer Aufwandmenge von 0,25 kg Wirkstoff je ha zeigten die Wirkstoffe 114, 124 und 98 eine gute herbizide Wirkung.

Bei der Prüfung der herbiziden Wirkung bei Nachauflaufanwendung im Gewächshaus bei einer Aufwandmenge von 0,25 kg Wirkstoff je ha zeigte der Wirkstoff 78 eine gute herbizide Wirkung.

Bei der selektiven herbiziden Wirkung bei Nachauflaufanwendung im Gewächshaus bei einer Aufwandmenge von 0,25 kg Wirkstoff je ha zeigte der Wirkstoff 187 eine gute herbizide Wirkung.

Bei der Prüfung der selektiven herbiziden Wirkung bei Nachauflaufanwendung im Gewächshaus bei einer Aufwandmenge von 0,25 kg Wirkstoff je ha zeigte der Wirkstoff 147 eine gute herbizide Wirkung.

Bei der Prüfung der selektiven herbiziden Wirkung bei Nachauflaufanwendung im Gewächshaus bei einer Aufwandmenge von 0,25 kg Wirkstoff je ha zeigten die Wirkstoffe 37 und 38 eine gute herbizide Wirkung.

**Patentansprüche für die Vertragsstaaten: BE, CH, DE, FR, GB, IT, LI, NL, SE**

1. Aralkylanilinderivat der allgemeinen Formel

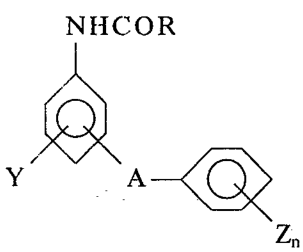

in der

R Methoxy, Ethoxy, Propoxy, sec. Butoxy, tert.-Butoxy, iso-Propoxy, Methylthio, Ethylthio, Cyclopropyl, Methyl, Ethyl, Propyl, tert.-Butyl,
A in 3- oder 4-Stellung zur NH-Gruppe einen gegebenenfalls durch Alkyl substituierten Alkylenrest mit 1 bis 10 C-Atomen
Y Wasserstoff, Alkyl, Halogen
Z Wasserstoff, Methyl, Ethyl, tert.-Butyl, Methoxy, Ethoxy, Hydroxy, Halogen, Trifluormethyl oder Phenyl oder eine $C_4H_4$-Kette, die mit dem Benzolring zu einem Naphthylring kondensiert ist.
n die Zahlen 1 bis 4

bedeutet, außer der Verbindung N-(4-(alpha-Methylbenzyl)-phenyl)-carbaminsäure-ethylester.

2. Aralkylanilinderivat gemäß Anspruch 1, dadurch gekennzeichnet, daß R Methoxy, A Propylen und Z Wasserstoff bedeutet.

3. Herbizid, enthaltend ein Aralkylanilinderivat gemäß Anspruch 1.

4. Herbizid, enthaltend einen festen oder flüssigen Trägerstoff und ein Aralkylanilinderivat gemäß Anspruch 1.

5. Verfahren zur Bekämpfung von unerwünschten Pflanzen, dadurch gekennzeichnet, daß man die Pflanzen oder den Boden behandelt mit einem Aralkylanilinderivat gemäß Anspruch 1.

**Patentansprüche für den Vertragsstaat: AT**

1. Herbizid, enthaltend ein Aralkylanilinderivat der allgemeinen Formel

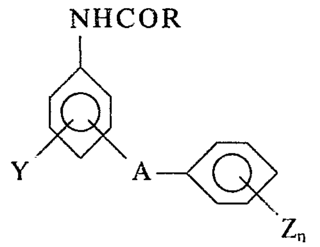

in der

R Methoxy, Ethoxy, Propoxy, sec. Butoxy, tert.-Butoxy, iso-Propoxy, Methylthio, Ethylthio, Cyclopropyl, Methyl, Ethyl, Propyl, tert.-Butyl
A in 3- oder 4-Stellung zur NH-Gruppe einen gegebenenfalls durch Alkyl substituierten Alkylenrest mit 1 bis 10 C-Atomen
Y Wasserstoff, Alkyl, Halogen
Z Wasserstoff, Methyl, Ethyl, tert.-Butyl, Methoxy, Ethoxy, Hydroxy, Halogen, Trifluormethyl oder Phenyl
n die Zahlen 1 bis 4

bedeutet.

2. Herbizid gemäß Anspruch 1, dadurch gekennzeichnet, daß R Methoxy, A Propylen und Z Wasserstoff bedeutet.

3. Herbizid, enthaltend einen festen oder flüssigen Trägerstoff und ein Aralkylanilinderivat gemäß Anspruch 1.

4. Verfahren zur Bekämpfung von unerwünschten Pflanzen, dadurch gekennzeichnet, daß man die Pflanzen oder den Boden behandelt mit einem Aralkylanilinderivat gemäß Anspruch 1.

**Claims for the contracting states BE, CH, LI, DE, FR, GB, IT, NL, SE**

1. An aralkylaniline derivative of the general formula

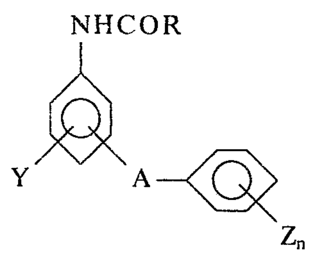

where

R is methoxy, ethoxy, propoxy, sec.-butoxy, tert.-butoxy, iso-propoxy, methylthio, ethylthio, cyclopropyl, methyl, ethyl, propyl, tert.-butyl,
A in the 3- or 4-position to the NH group is unsubstituted or alkyl-substituted alkylene of 1 to 10 carbon atoms,
Y is hydrogen, alkyl or halogen,
Z is hydrogen, methyl, ethyl, tert.-butyl, methoxy, ethoxy, hydroxy, halogen, trifluoromethyl, phenyl or a $C_4H_4$-chain which is fused to the benzene radical to form a naphthyl ring, and
n is an integer from 1 to 4,

except to compound ethyl N-(4-(alpha-methylbenzyl)-phenyl)-carbamate.

2. An aralkylaniline derivative as claimed in claim 1, wherein R is methoxy, A is propylene and Z is hydrogen.

3. A herbicide containing an aralkylaniline derivative as claimed in claim 1.

4. A herbicide containing a solid or liquid carrier and an aralkylaniline derivative as claimed in claim 1.

5. A process for controlling the growth of unwanted plants, wherein the plants or the soil are treated with an aralkylaniline derivative as claimed in claim 1.

**Claims for the contracting states AT**

1. An aralkylaniline derivative of the general formula

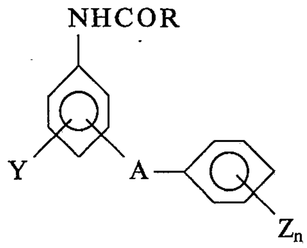

where

R is methoxy, ethoxy, propoxy, sec.-butoxy, tert.-butoxy, iso-propoxy, methylthio, ethylthio, cyclopropyl, methyl, ethyl, propyl, tert.-butyl,
A in the 3- or 4-position to the NH group is unsubstituted or alkyl-substituted alkylene of 1 to 10 carbon atoms,
Y is hydrogen, alkyl or halogen,
Z is hydrogen, methyl, ethyl, tert.-butyl, methoxy, ethoxy, hydroxy, halogen, trifluoromethyl or phenyl, and
n is an integer from 1 to 4.

2. An aralkylaniline derivative as claimed in claim 1, wherein R is methoxy, A is propylene and Z is hydrogen.
3. A herbicide containing a solid or liquid carrier and an aralkylaniline derivative as claimed in claim 1.
4. A process for controlling the growth of unwanted plants, wherein the plants or the soil are treated with an aralkylaniline derivative as claimed in claim 1.

**Revendications pour les Etats contractants: BE, CH, LI, DE, FR, GB, IT, NL, SE**

1. Dérivé d'aralkylaniline de formule générale

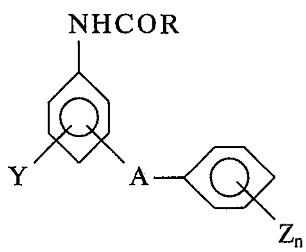

dans laquelle

R représente méthoxy, éthoxy, propoxy, sec.butoxy, tert.-butoxy, iso-propoxy, méthylthio, éthylthio, cyclopropyl, méthyle, éthyle, propyle, tert.-butyle,
A un reste alkylène de 1 à 10 atomes C, éventuellement substitué par alkyle, en position 3 ou 4 par rapport au groupe NH,
Y hydrogène, alkyle, halogène,
Z hydrogène, méthyle, éthyle, tert.-butyle, méthoxy, éthoxy, hydroxy, halogène, trifluorométhyle ou phényle ou une chaîne $C_4H_4$, qui est transformée, par condensation avec le noyau benzène, en un noyau naphtyle,
n représente les nombres 1 à 4,

outre le composé N-(4-(alpha-méthylbenzyl)-phényl)-carbamate d'éthyle.
2. Dérivé d'aralkylaniline selon la revendication 1, caractérisé par le fait que R représente méthoxy, A représente propylène et Z représente hydrogène.
3. Herbicide contenant un dérivé d'aralkylaniline selon la revendication 1.
4. Herbicide contenant un véhicule solide ou liquide et un dérivé d'aralkylaniline selon la revendication 1.
5. Procédé de lutte contre les plantes indésirables, caractérisé par le fait qu'on traite les plantes ou le sol avec un dérivé d'aralkylaniline selon la revendication 1.

**Revendications pour l'Etat contractant: AT**

1. Herbicide contenant un dérivé d'aralkylaniline de formule générale

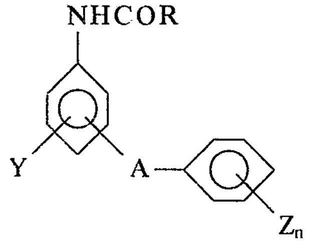

dans laquelle

R représente méthoxy, éthoxy, propoxy, sec.butoxy, tert.-butoxy, iso-propoxy, méthylthio, éthylthio, cyclopropyl, méthyle, éthyle, propyle, tert.-butyle,
A un reste alkylène de 1 à 10 atomes C, éventuellement substitué par alkyle, en position 3 ou 4 par rapport au groupe NH,
Y hydrogène, alkyle, halogène,
Z hydrogène, méthyle, éthyle, tert.-butyle, méthoxy, éthoxy, hydroxy, halogène, trifluorométhyle, ou phényle ou une chaîne $C_4H_4$, qui est transformée, par condensation avec le noyau benzène, en un noyau naphtyle,
n représente les nombres 1 à 4.

2. Herbicide selon la revendication 1, caractérisé par le fait que R représente méthoxy, A représente propylène et Z représente hydrogène.

3. Herbicide contenant un véhicule solide ou liquide et un dérivé d'aralkylaniline selon la revendication 1.

4. Procédé de lutte contre les plantes indésirables, caractérisé par le fait qu'on traite les plantes ou le sol avec un dérivé d'aralkylaniline selon la revendication 1.